# EUROPÄISCHE PATENTANMELDUNG

(11) **EP 2 620 146 A1**
(43) Veröffentlichungstag der Anmeldung: **31.07.2013**
(21) Anmeldenummer: 13152804.4
(22) Anmeldetag: 28.01.2013
(51) Int. Cl.: A61K 31/192, A61K 9/00, A61P 17/00

(54) **Ibuprofen zur Behandlung von aktinischer Keratose**

(30) Priorität: 30.01.2012 EP 12153082
(71) Anmelder: Dolorgiet GmbH & Co. KG, 53757 Sankt Augustin (DE)
(72) Erfinder: Warnecke, Jürgen, 25560 Oldenborstel (DE)
(74) Vertreter: von Kreisler Selting Werner

(57) **Zusammenfassung**

Ibuprofen zur Verwendung in der Vorbeugung und Behandlung von aktinischer Keratose.

## Beschreibung

Die vorliegende Erfindung betrifft Zusammensetzungen zur Behandlung von aktinischer Keratose.

Die aktinische Keratose ist eine durch langjährige, intensive Einwirkung von Sonnenlicht oder UV-Strahlung verursachte chronische Schädigung der verhornten Oberhaut. Aktinische Keratose ist eine fakultative Präkanzerose und kann in ein Plattenepithelkarzinom übergehen.

Die aktinische Keratose tritt vor allem bei Menschen in der zweiten Lebenshälfte auf, wobei Stellen, die dem Sonnenlicht ungeschützt ausgesetzt waren, besonders häufig betroffen sind, beispielsweise Gesicht, Handrücken, Stirn, Nase oder Ohr. Neben dem Menschen sind auch Tiere, wie beispielsweise Hunde und Katzen, an Stellen geringer Behaarung betroffen.

Typische Behandlungsformen umfassen eine operative Entfernung oder eine Behandlung mit beispielsweise Imiquimod, 5-Fluorouracil, Hyaluronsäure mit Diclofenac oder photodynamische Therapie mit 5-Aminolävulinsäure.

Insbesondere im Bereich des Gesichtes oder bei großflächigeren Erkrankungen können chirurgische Verfahren kosmetisch unerwünscht sein. Daher wird insbesondere im Gesichtsbereich bevorzugt mit Gelen und Cremes topisch behandelt. Dabei ist aktuell die Behandlung mit einer Kombination aus Diclofenac/Hyaluronsäure am verträglichsten.

Aufgabe der vorliegenden Erfindung war es, weitere Behandlungsmöglichkeiten für die aktinische Keratose bereitzustellen.

Gelöst wird die Aufgabe durch die Verwendung von Ibuprofen in der Vorbeugung und Behandlung von aktinischer Keratose.

Ibuprofen ist ein seit vielen Jahren eingesetzter Wirkstoff bei der Behandlung von Arthrose, Sportverletzungen sowie rheumatischen Erkrankungen. Neben einer systemischen Anwendung sind auch entsprechende topische Anwendungsformen bekannt.

Für eine generelle systemische Wirkung sind Plasmaspiegel im Bereich von 10 µg/ml oder mehr notwendig. Bei topischer Anwendung werden nur Plasmaspiegel von etwa 2 µg/ml erreicht. Bei einer topischen Anwendung kommt es daher nicht zu systemischen Effekten und auch die Nebenwirkungen sind äußerst gering.

Bevorzugt wird daher erfindungsgemäß Ibuprofen in einer topischen Zubereitung eingesetzt.

Besonders geeignete Zubereitungen sind Salben, Cremes und Gele. Eine Creme ist eine Emulsion einer wässrigen in einer liphophilen Phase; ein Gel ein disperses System aus einer festen und einer flüssigen Phase.

Als besonders geeignet haben sich Konzentrationen von Ibuprofen in einer topischen Zubereitung von 1 bis 20 Gew.-% erwiesen, wobei Konzentrationen von 5 bis 10 Gew.-% besonders bevorzugt sind. Bevorzugt beträgt die Konzentration mehr als 5, oder mindestens 6 oder 7 Gew.-%. Die Konzentration liegt bevorzugt bei 10 Gew.-% oder unterhalb von 10% Gew.-%, beispielsweise bis zu 9 Gew.-% oder bis zu 8 Gew.-%. Der Bereich von 5 bis 10 Gew.-% oder 6 bis 8 Gew.-% ist insbesondere für Gelzubereitungen bevorzugt.

In einer Ausführungsform enthält die Zubereitung zusätzlich Penetrationsverbesserer, die die Durchdringung der Haut erleichtern. Solche Penetrationsverbesserer sind dem Fachmann grundsätzlich bekannt; auch aus der Herstellung von Ibuprofen-haltigen topischen Zubereitungen zur Behandlung von beispielsweise Sportverletzungen.

Besonders geeignete Zubereitungen enthalten als Penetrationsverbesserer Dimethylisosorbit, Poloxamere, Laurocapram, Pyrolidone, Dimethylsulfoxid, Ölsäure, Ethylenglykole und Derivate und Mischungen davon.

In einer Ausführungsform der Erfindung ist Ibuprofen der einzige aktive Wirkstoff in der Zubereitung. Selbstverständlich benötigt Ibuprofen je nach Applikationsform Hilfsstoffe zur Herstellung eines verabreichbaren Arzneimittels. In dieser Ausführungsform sind jedoch keine weiteren aktiven Wirkstoffe enthalten.

In einer Ausführungsform ist insbesondere keine Hyaluronsäure enthalten, die Hilfsstoff oder Wirkstoff sein könnte.

In einer anderen Ausführungsform ist zusätzlich zu Ibuprofen Diclofenac enthalten. Bevorzugte Mengen an Diclofenac liegen ebenfalls im Bereich von 1 bis 20 Gew.-%, wobei Konzentrationen von 3 bis 10 Gew.-% bevorzugt sind. Bevorzugt sollte die gesamte Konzentration an Ibuprofen und Diclofenac im Bereich von 1 bis 20, bevorzugt 5 bis 10 Gew.-% liegen.

Eine bevorzugte Gel-Formulierung (Mikroemulsionsgel) enthält:
- 5 bis 10 g Ibuprofen,
- 10 bis 25 g Isopropanol,
- 5 bis 10 g Dimethylisosorbid,
- 6 bis 18 g Poloxamer,
- 1 bis 4 g mittelkettige Triglyceride,
- Duftstoffe,
- Wasser auf 100 g.

Ein weiteres bevorzugtes Gel enthält:
- 5 bis 10 g Ibuprofen,
- 30 bis 60 g Ethanol 96%ig,
- 5 bis 20 g Dimethylisosorbid,
- 1 bis 4 g Hydroxypropylcellulose,
- 0,75 bis 4 g Kaliumhydroxid,
- Wasser auf 100 g.

Die Erfindung wird durch die folgenden Beispiele näher erläutert.

### Beispiele

In einer vorläufigen Studie wurde die Behandlung der aktinischen Keratose mit Ibuprofen mit einer Behandlung mit Diclofenac verglichen. Die Durchführung der Studie erfolgte nach den einschlägigen Kriterien und wurde von der Ethikkommission der Dermatologischen Gesellschaft der Slowakei positiv votiert.

In die Studie wurden 24 Patienten eingeschlossen, die fünf oder mehr Läsionen im Bereich des Kopfes, des Gesichts, der Arme oder der Hände aufwiesen.

12 der Patienten wurden mit Ibuprofen 5% Gel und die anderen mit 3% Diclofenac Gel behandelt. Die Anwendung erfolgte zweimal täglich. Der Behandlungserfolg wurde nach 30, 60 und 90 Tagen beurteilt.

Bei Aufnahme in die Studie wurde die Schwere der Läsionen nach dem so genannten BSI-Score (Baseline Severity Index) zwischen 0 und 3 beurteilt. Es wurden ausschließlich Patienten mit dem BSI-Score 1 und 2 in die Studie aufgenommen.

### BSI-Score (Baseline Severity Index Score 1 - 3)

0 = keine aktinische Keratose (AKS) sichtbar
1 = deutlich sichtbare Läsion
2 = viele kleine, moderat dicke Läsionen oder wenige große, schuppige Läsionen sichtbar
3 = viele dicke, hypertrophe Läsionen, die deutlich sichtbar und fühlbar sind mit klarer Begrenzung.

Hauptzielparameter im Rahmen der Wirksamkeit war die Gesamtzahl der Läsionen im Behandlungsbereich sowie der IGII-Score (Investigator Global Improvement Index) zwischen -2 und +4. Er wurde immer im Vergleich zur Vorvisite ab Visite 1 beurteilt.

**Tabelle 1: Mittlere Anzahl der Läsionen zum Start der Studie**

| | **Ibuprofen** | **Diclofenac** |
|---|---|---|
| Stirn | 4,6 | 7,1 |
| Gesicht | 5,7 | 7 |
| Kopfhaut | 6,3 | 15,8 |
| Hände | 18,5 | 18,1 |

**Tabelle 2: Mittlere Anzahl der Läsionen nach 90tägiger Behandlung**

| | **Ibuprofen** | **Diclofenac** |
|---|---|---|
| Stirn | 4 | 5,3 |
| Gesicht | 4,8 | 5,5 |
| Kopfhaut | 6 | 11,5 |
| Hände | 16,9 | 17,1 |

Nach 90-tägiger Behandlung verminderte sich die mittlere Läsionszahl in der Ibuprofen-Gruppe von 35,1 auf 31,7 und in der Diclofenac-Gruppe von 48 auf 39,4. Die Schwere der Läsionen blieb während der 90-tägigen Behandlung praktisch unverändert. Lediglich auf der Stirn kam es zu einer geringen Reduktion des BSI-Scores. Keiner der Patienten entwickelte während der Behandlung neue Läsionen.

### IGII (Investigator Global Improvement Index)

| | |
|---|---|
| - 2 | signifikante Verschlechterung |
| - 1 | leichte Verschlechterung |
| 0 | unverändert |
| 1 | leichte Verbesserung |
| 2 | Verbesserung |
| 3 | signifikante Verbesserung |
| 4 | komplette Heilung |

Der IGII wurde jeweils zwischen zwei Besuchen gemessen. Er kann einen Wert von -2 (starke Verschlechterung) bis +4 (Heilung) annehmen und veränderte sich während der 90-tägigen Behandlung wie folgt:

**Tabelle 3: Investigator Global Improvement Index zum Start der Studie (V0) und nach 90tägiger Behandlung (V3)**

| | **Ibuprofen** | | **Diclofenac** | |
|---|---|---|---|---|
| | **V0** | **V3** | **V0** | **V3** |
| Stirn | 0 | 1,33 | 0 | 1,55 |
| Gesicht | 0 | 1,38 | 0 | 1,7 |
| Kopfhaut | 0 | 1 | 0 | 1,75 |
| Hand | 0 | 1 | 0 | 1 |

### Ergebnis

Es kam in beiden Gruppen zu einer Verringerung der Läsionszahl, die zwar nur geringfügig ausfiel, allerdings in Kombination mit der positiven Veränderung des IGII-Scores eine in etwa vergleichbare Wirksamkeit beider Behandlungen (Ibuprofen und Diclofenac) unterstreicht. Die Unterschiede in der Reduktion der Läsionen im Vergleich von Ibuprofen und Diclofenac gehen im Wesentlichen auf die Anzahl der Läsionen zum Start der Studie zurück. Während der Untersuchung zeigten sich keine unerwünschten Nebenwirkungen. Die Therapie wurde gut vertragen.

In einer weiteren Studie wurden sechs Patienten mit Ibuprofen 10% Gel und weitere sechs Patienten mit 3% Diclofenac Gel behandelt. Das Studiendesign entsprach dem Design der ersten Studie.

Die Gesamtzahl der Läsionen in der Ibuprofen-Gruppe veränderte sich von 18 zu Beginn der Behandlung auf 3,7 am Ende der 90-tägigen Behandlungszeit. In der Diclofenac-Gruppe waren es zum Start 36,8 Läsionen, die sich zum Ende der Behandlung auf 19,2 Läsionen im Mittel veränderten.

**Tabelle 4: Mittlere Anzahl der Läsionen zum Start der Studie**

| | **Ibuprofen** | **Diclofenac** |
|---|---|---|
| Stirn | 3,3 | 7,5 |
| Gesicht | 5,2 | 12,3 |
| Kopfhaut | Keine Bewertung, da nur 1 Patient Läsionen zeigte. | |
| Hände | 9,5 | 17 |

**Tabelle 5: Mittlere Anzahl der Läsionen nach 90tägiger Behandlung**

| | **Ibuprofen** | **Diclofenac** |
|---|---|---|
| Stirn | 0,3 | 4,2 |
| Gesicht | 0,7 | 6,2 |
| Kopfhaut | Keine Bewertung, da nur 1 Patient Läsionen zeigte. | |
| Hände | 2,7 | 8,8 |

Der IGII lag im Durchschnitt am Ende der Behandlungszeit in der Ibuprofen-Gruppe bei 3,7 (die Läsionen waren also fast vollständig abgeheilt) und in der Diclofenac-Gruppe bei 2,29, was einem moderaten Heilungserfolg entspricht.

**Tabelle 6: Investigator Global Improvement Index zum Start der Studie (V0) und nach 90tägiger Behandlung (V3)**

| | **Ibuprofen** | | **Diclofenac** | |
|---|---|---|---|---|
| | V0 | V3 | V0 | V3 |
| Stirn | 0 | 3,8 | 0 | 2,3 |
| Gesicht | 0 | 3,7 | 0 | 2,17 |
| Kopfhaut | Keine Bewertung, da nur 1 Patient Läsionen zeigte. | | | |
| Hand | 0 | 3,6 | 0 | 2,4 |

### Ergebnis

Auch wenn man die geringe Patientenzahl in der 2. Studie betrachtet, so zeigt doch der Behandlungserfolg in der Ibuprofen-10%-Gruppe, dass die Formulierung eine ausgeprägte Wirksamkeit im Bereich der Behandlung leichter und mittelschwerer Keratose-Läsionen besitzt. Die Therapieergebnisse der Diclofenac-Behandlung fallen ebenfalls gut aus, obwohl bedingt durch die hohe Anfangsläsionszahl noch keine Gesamtheilung zu verzeichnen war. Es bleibt noch anzumerken, dass drei Patienten der Ibuprofen-Gruppe am Ende der 90-tägigen Behandlung vollständig von den optisch sichtbaren Läsionen befreit waren. Die Verträglichkeit in beiden Behandlungsgruppen war sehr gut.

## Patentansprüche

1. Ibuprofen zur Verwendung in der Vorbeugung und Behandlung von aktinischer Keratose.

2. Ibuprofen zur Verwendung nach Anspruch 1, **dadurch gekennzeichnet, dass** es in einer topischen Zubereitung vorliegt.

3. Ibuprofen zur Verwendung nach Anspruch 2, **dadurch gekennzeichnet, dass** es in Form einer Salbe, einer Creme oder eines Gels vorliegt.

4. Ibuprofen zur Verwendung nach einem der Ansprüche 2 oder 3, **dadurch gekennzeichnet, dass** die Konzentration des Ibuprofen 1 bis 20 Gew.-% beträgt.

5. Ibuprofen zur Verwendung nach Anspruch 4, **dadurch gekennzeichnet, dass** die Konzentration 5 bis 10 Gew.-% beträgt.

6. Ibuprofen zur Verwendung nach Anspruch 5, **dadurch gekennzeichnet, dass** die Konzentration 6 bis 8 Gew.-% beträgt.

7. Ibuprofen zur Verwendung nach einem der Ansprüche 2 bis 6, **dadurch gekennzeichnet, dass** Penetrationsverbesserer ausgewählt aus der Gruppe bestehend aus Dimethylisosorbid, Poloxameren, Laurocapram, Pyrolidone, Dimethylsulfoxid, Ölsäure, Ethylenglykole und Derivate und Mischungen davon enthalten sind.

8. Ibuprofen zur Verwendung nach einem der Ansprüche 1 bis 7, **dadurch gekennzeichnet, dass** Ibuprofen der einzige aktive Wirkstoff ist.

9. Ibuprofen zur Verwendung nach einem der Ansprüche 1 bis 7, **dadurch gekennzeichnet, dass** zusätzlich Diclofenac enthalten ist.

10. Ibuprofen zur Verwendung nach einem der Ansprüche 2 bis 9, **dadurch gekennzeichnet, dass** die Zubereitung keine Hyaluronsäure umfasst.

11. Pharmazeutisches Präparat enthaltend 6 bis 8 Gew.-% Ibuprofen und 2 bis 15 Gew.-% Penetrationsverbesserer.

12. Pharmazeutisches Präparat nach Anspruch 11, **dadurch gekennzeichnet, dass** der Penetrationsverbesserer Dimethylisosorbid ist.

13. Pharmazeutisches Präparat nach Anspruch 11 oder 12 in Form eines Gels.
